(19) Europäisches Patentamt
European Patent Office
Office européen des brevets

(11) **EP 3 263 164 B1**

(12) **EUROPÄISCHE PATENTSCHRIFT**

(45) Veröffentlichungstag und Bekanntmachung des Hinweises auf die Patenterteilung:
**11.03.2020 Patentblatt 2020/11**

(21) Anmeldenummer: **17001014.4**

(22) Anmeldetag: **15.06.2017**

(51) Int Cl.:
*A61M 16/00* *(2006.01)*　*A61M 16/10* *(2006.01)*
*A61B 5/08* *(2006.01)*

(54) **BEATMUNGSGERÄT ZUR APAP-BEATMUNG MIT OSZILLATORISCHEM DRUCK**

VENTILATOR FOR APAP-VENTILATION WITH OSCILLATING PRESSURE

APPAREIL DE RESPIRATION POUR VENTILATION APAP AVEC PRESSION OSCILLATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.06.2016 DE 102016007303**

(43) Veröffentlichungstag der Anmeldung:
**03.01.2018 Patentblatt 2018/01**

(73) Patentinhaber: **Löwenstein Medical Technology S.A.**
**2557 Luxembourg (LU)**

(72) Erfinder:
• **Scheerer, Mario**
**76227 Karlsruhe (DE)**
• **Sartor, Jürgen**
**76185 Karlsruhe (DE)**
• **Landskron, Florian**
**76187 Karlsruhe (DE)**
• **Schwaibold, Matthias**
**76228 Karlsruhe (DE)**

(74) Vertreter: **Marx, Thomas**
**Löwenstein Medical Technology GmbH + Co. KG**
**IP Management**
**Kronsaalsweg 40**
**22525 Hamburg (DE)**

(56) Entgegenhaltungen:
EP-A1- 0 651 971　　EP-A1- 1 393 767
WO-A1-97/10019　　WO-A1-2006/047826
WO-A1-2008/025080　　WO-A1-2013/042007
WO-A1-2013/173219　　DE-U1-202004 000 848
US-A1- 2012 157 794　　US-B1- 6 968 842

**Beschreibung**

Die EP 0 705 615 beschreibt ein Verfahren zur Steuerung eines Beatmungsgerätes dadurch, dass mit der Oszilloresistometrie die oszillatorische Druckamplitude, die dem Atemwiderstand des Patienten entspricht, kontinuierlich gemessen wird, wobei nach dem Bestimmen des individuellen Atemwiderstandswertes (Basiswert der Druckamplitude) bei Abweichungen von diesem Wert dem Patienten Atemgas unter Druck zugeführt und die Gaszufuhr beendet wird, sobald der Basiswert wieder oder annähernd erreicht ist. Bei diesem Verfahren zur Steuerung und Regelung eines Beatmungsgerätes zur Therapie von Schlafapnoe-Patienten wird dieses Gerät nur dann aktiviert, d. h. es wird dem Patienten nur dann Atemgas unter erhöhtem Druck zugeführt, wenn durch eine Apnoe die Atemtätigkeit des Patienten gestört ist oder sich der Atemwiderstand - und somit auch die gemessene oszillatorische Druckamplitude - durch eine entstehende Apnoe ändert. Eine Störung der Atemtätigkeit des Patienten ist von einer Veränderung des Atemwiderstandes des Patienten begleitet. Dieser Widerstand wird ohne das Befinden des Patienten zu beeinflussen, auf einfache Weise zuverlässig und reproduzierbar über die Veränderungen der oszillatorischen Druckamplitude bestimmt. Im Verlauf einer beginnenden Apnoe bewirken Verengungen oder Erweiterungen der Atemwege eines Patienten Änderungen im Phasenwinkel der oszillatorischen Druckamplitude und des Atemflusses im Vergleich zu den entsprechenden Basiswerten bei normalem oder ungestörtem Atemhub.

Das Beatmungsgerät zur Therapie der Schlafapnoe weist eine Atemmaske auf, die über einen Atemschlauch mit einer als Lüfter ausgebildeten Druckgasquelle verbunden ist. Im Atemschlauch sind vor der Atemmaske die Sensoren einer Einrichtung zum Erzeugen, Messen und Filtern der oszillatorischen Druckamplitude nach dem ORM-Prinzip sowie des Phasenwinkels vorgesehen. Von einer Steuer-Regeleinrichtung, die mit der Einrichtung verbunden und auf den individuellen Basiswert der dem Atemwiderstand des Patienten entsprechenden oszillatorischen Druckamplitude einstellbar ist, wird die Druckgasquelle 3 so aktiviert, dass dem Patienten Atemgas bei signifikanten Veränderungen des Phasenwinkels zugeführt wird. Die Einrichtung 5 umfasst auch eine ventillose Membranpumpe, mit der dem Atemfluss ein sinusförmiger Wechselfluss überlagert wird.

Die EP 0 821 977 betrifft ein Verfahren zur Steuerung eines Beatmungsgerätes zur Therapie der Schlafapnoe sowie ein

Beatmungsgerät zur Durchführung des Verfahrens, wobei der Phasenwinkel (die zeitliche Differenz zwischen Atemfluss und Atemdruck) und die dem Atemwiderstand entsprechende Druckamplitude gemessen sowie der individuelle Atemwiderstandswert (Basiswert) der Druckamplitude eines Patienten mit Hilfe der Oszilloresistometrie bestimmt und der Atemgasdruck in Abhängigkeit von diesen Größen geregelt werden.

Zur Durchführung einer Ermittlung des Strömungsvolumens ist es beispielsweise bekannt, Sensoren mit temperierbaren Hitzdrähten zu verwenden, die mit temperaturabhängigen Widerständen versehen sind. Bei einer Beheizung eines der Widerstandsdrähte kann im Bereich eines weiteren Hitzdrahtes eine Temperaturveränderung erfasst werden, die vom jeweiligen Strömungsvolumen abhängig ist. Ebenfalls ist es bereits bekannt, über dem Strömungsvolumen ausgesetzte Messbrücken mit temperaturabhängigen Widerständen das Strömungsvolumen zu erfassen, da auch hier Abkühleffekte vom jeweiligen Strömungsvolumen abhängig sind. Die bekannten Sensoren erweisen sich jedoch als relativ aufwendig und teuer.

Nachteilig ist, dass die Vorrichtung eine Pumpe erfordert, mit der ein oszillierender Wechselfluss erzeugt wird. Nachteilig ist ebenfalls, dass die Signale der Oszilloresistometrie mittels eines Fluss- und/oder Drucksensors aufgezeichnet werden müssen.

Die WO 2013042007 offenbart eine autoCPAP-Beatmungsvorrichtung, mit einer Steuereinrichtung und einem Beatmungsgebläse und einem Drucksensor, wobei die Steuereinrichtung einen Controller zur Erzeugung eines ersten Steuersignals aufweist, welches die Drehzahl des Gebläses zur Erzeugung eines unter Druck stehenden Atemgasflusses veranlasst, und wobei die Steuereinrichtung einen Controller zur Erzeugung eines periodisch veränderlichen Steuersignals aufweist, welcher das Gebläse derart ansteuert, dass die Drehzahl des Gebläses mit einer Frequenz im Bereich 1 - 20 Hz oszillierend schwankt.
Die EP 0651971 und die WO 2008025080 beschreiben vergleichbare autoCPAP-Beatmungsvorrichtungen.
Die EP 1393767 und die WO 2006047826 offenbaren ähnliche Beatmungsvorrichtungen, bei denen anhand von mathematischen Modellen und anhand von hinterlegten Werten oder Kennlinien Atemgasfluss und Atemgasvolumen rechnerisch bestimmt werden.

Die WO 9710019 und die US 2012157794 offenbaren jeweils autoCPAP-Beatmungsvorrichtungen, die Atemereignisse wie Apnoen erkennen.

Die Aufgabe der Erfindung besteht in der Bereitstellung einer autoCPAP-Beatmungsvorrichtung, die mittels der Oszilloresistometrie, bei geringem apparativen Aufwand, eine Erkennung und Typisierung von Atemereignissen ermöglicht, um Atemereignisse zu differenzieren und so die Therapie zu verbessern hilft.

In der Beschreibung der vorliegenden Erfindung werden die Begriffe Atemgasfluss und Fluss synonym verwendet.

Diese Aufgabe wird erfindungsgemäß gelöst durch eine autoCPAP-Beatmungsvorrichtung, mit einer Steuereinrichtung und einem Beatmungsgebläse und einem Drucksensor, wobei die Steuereinrichtung einen Controller zur Erzeugung eines ersten Steuersignals aufweist, welches die Drehzahl des Gebläses zur Erzeugung eines unter Druck stehenden Atemgasflusses veranlasst, und wobei die Steuereinrichtung einen Controller zur Erzeugung eines periodisch veränderlichen Steuersignals aufweist, welcher das Gebläse derart ansteuert, dass die Drehzahl des Gebläses mit einer Frequenz im Bereich 1 - 20 Hz oszillierend schwankt, zur Erzeugung eines einer FOT-Schwingung, wobei die Steuereinrichtung eine Sensoreinrichtung aufweist, die zumindest die momentane Drehzahl des Gebläses ermittelt und/oder den momentanen elektrischen Strom des Gebläses ermittelt und/oder die momentane elektrische Leistung des Gebläses ermittelt zur Bestimmung des van dem Gebläse erzeugten Atemgasflusses und/oder Atemgasvolumens unter Verwendung van in einem Speicher hinterlegten Kenndaten des Gebläses und vom Drucksensor bereitgestellten Druckwerten, wobei

- die autoCPAP-Beatmungsvorrichtung Sensormittel zur Erfassung eines ODS-Signals aufweist und dazu ausgebildet ist, das ODS-Signal an die Steuereinrichtung zu liefern,
- die autoCPAP-Beatmungsvorrichtung dazu ausgebildet ist, Ereignisse, die in der Atmung beziehungsweise während der Beatmung auftreten, zu erkennen und typisieren, wobei die Steuereinrichtung die Signale Druck und Fluss und ODS mittels geeigneter Algorithmen analysiert, sodass charakteristische Signalverlaufe erkannt und als ein Ereignis typisiert werden, wobei
- eine obstruktive Apnoe (oA) erkannt wird, wenn für mindestens zwei Atemzuge ein stark reduziertes Flussvolumen erkannt wird und/oder ein ODS-Anstieg erfolgt und wobei
- eine obstruktive Hypopnoe (oH) durch ein reduziertes Flussvolumen für zwei aufeinanderfolgende Inspirationen erkannt wird.

Die Aufgabe wird ergänzend dadurch gelöst, dass sowohl eine Drehzahl des Motors als auch ein Druck im Bereich des Strömungsvolumens messtechnisch erfasst sowie der Steuereinrichtung zugeführt werden und dass das Strömungsvolumen von der Steuereinrichtung durch eine rechnerische Verknüpfung der Messwerte für den Druck und die Drehzahl ermittelt wird.

Die erfindungsgemäße Sensoreinrichtung ist bevorzugt kein gegenständlich vorhandener Fluss-Sensor. Die erfindungsgemäße Sensoreinrichtung ist bevorzugt eine Berechnungsvorschrift oder ein Algorithmus, die zumindest die momentane Drehzahl des Gebläses ermittelt und/oder den momentanen elektrischen Strom des Gebläses ermittelt und/oder die momentane elektrische Leistung des Gebläses ermittelt zur Bestimmung des von dem Gebläse erzeugten Atemgasflusses und/oder Atemgasvolumens unter Verwendung von in einem Speicher hinterlegten Kenndaten des Gebläses und vom Drucksensor bereitgestellten Druckwerten.

Erfindungsgemäß kann durch die Verwendung der Sensoreinrichtung ein Fluss-Sensor (der zumindest 20 € kostet) eingespart werden.

Weitere Aufgabe der vorliegenden Erfindung ist es, eine Vorrichtung der einleitend genannten Art derart zu konstruieren, dass eine hohe Messgenauigkeit bei Verwendung einer einfachen Sensoreinrichtung erreicht wird.

Diese Aufgabe wird erfindungsgemäß dadurch gelöst, dass der Motor mit einer Sensoreinrichtung zur Drehzahlerfassung versehen und im Bereich des Strömungsvolumens ein Sensor zur Druckmessung angeordnet ist, dass der Sensor und die Sensoreinrichtung an die Steuereinrichtung angeschlossen sind und dass die Steuereinrichtung mit einer Recheneinheit zur Bestimmung des Strömungsvolumens und/oder des Flusses durch eine rechnerische Verknüpfung der Messwerte für den Druck und die Drehzahl versehen ist.

Die messtechnische Erfassung der Drehzahl des Motors sowie die messtechnische Erfassung des Druckes im Bereich des Strömungsvolumens können mit konstruktiv einfachen Sensoren preiswert und mit hoher Genauigkeit erfolgen. Durch die rechnerische Auswertung dieser Messgrößen im Bereich der Steuereinrichtung ist es möglich, unter Berücksichtigung eines mathematischen Modells der strömungstechnischen Eigenschaften der Gesamtanordnung das jeweilige Strömungsvolumen zu ermitteln. Die Berechnung des Strömungsvolumens aus den gemessenen Parametern erfolgt nach vergleichsweise einfachen Berechnungsvorschriften, so dass die Umrechnung beispielsweise unter Verwendung eines einfachen Mikroprozessors durchgeführt werden kann. Alternativ ist es aber auch möglich, eine analoge Ermittlung mit Hilfe von Bauelementen durchzuführen, die geeignete Kennlinien besitzen.

Eine Ermittlung eines aktuellen Strömungsvolumens wird dadurch unterstützt, dass die rechnerische Verknüpfung unter Berücksichtigung einer Gerätecharakteristik durchgeführt wird, die durch ein Kennlinienfeld definiert wird.

Zur Ermöglichung einer Ermittlung des Strömungsvolumens mit geringem Zeitaufwand wird vorgeschlagen, dass das Kennlinienfeld vor einer Erfassung des Strömungsvolumens messtechnisch aufgenommen wird und im Beatmungsgerät abgespeichert ist.

Eine weitere Verkürzung des Zeitaufwandes kann dadurch erfolgen, dass eine tatsächliche physikalische Abhängigkeit zwischen dem Strömungsvolumen und einem im Bereich des Strömungsvolumens herrschenden Druckes durch eine gemischt quadratische Gleichung angenähert wird.

Zur Bereitstellung einfacher Berechnungsvorschriften wird vorgeschlagen, dass eine Parametrisierung des Kennlinienfeldes durchgeführt wird.

*weiter auf der ursprünglichen Seite 5 ab Zeile 6*

Alternativ oder ergänzend kann ein hinterlegter gerätetypischer Kompensationsparameter abgerufen und genutzt werden um den ermittelten Fluss zumindest zeitweise anzupassen.

Der auf diese Weise ermittelte Gesamtfluss kann weiter separiert werden in Masken-Spülfluss, ungewollten Leckagefluss und respiratorischen Fluss.

Auf Basis des so ermittelten respiratorischen Flusses erfolgt die FOT-Auswertung. Die FOT-Schwingung wird mit dem Beatmungsgebläse erzeugt. Dazu wird auf den Therapie-Solldruck die 1 - 30 Hz, bevorzugt eine 1 - 20 Hz-Schwingung aufmoduliert und vom Druckregler mit ausgeregelt. Die Schwingung kann auch im Bereich 2 - 10 Hz liegen.

Zur Erzeugung der FOT-Schwingung wird der Solldruck oszillierend vom Controller geändert. Der Druckregler folgt mit dem Ist-Druck. Bei der hohen Frequenz ist das jedoch auch fehlerbehaftet. Je nach Resistance und Compliance von Lunge, Schlauch, Maske, Atemwegen oder anderen Faktoren oszilliert der Ist-Druck also mit einer individuell etwas abweichenden Amplitude. Hier kommt zum ersten Mal der berechnete Fluss ins Spiel. Der Druckregler schätzt den Druckabfall über den Schlauch und sonstiges Zubehör anhand des berechneten Flusses und versucht diesen zu kompensieren. Erst dadurch ist es für den Druckregler möglich, die Vorgabe des Controllers umzusetzen.

Zur Erzeugung der FOT-Schwingung kann alternativ auch nur die Drehzahl mit einer festen Amplitude oszillieren.

Die Durchgängigkeit der Atemwege wird anhand des Quotienten der Amplitude aus berechnetem Fluss und Druck ermittelt. Es wird also ermittelt, wie viel Fluss pro Druck erzeugt werden kann. Sind die Atemwege offen, ist es viel Fluss. Sind sie geschlossen, ist es wenig Fluss. Die Schwelle liegt bei 12,5 l/min / hPa, falls keine Leckage vorliegt. Je höher die Leckage, desto mehr verschiebt sich der Schwellwert, da man bei einem undichten System ohnehin mehr Fluss pro Druckschwankung erzeugen kann. Dabei ist beides relevant: gewollte Leckage (Spülfluss) und ungewollte Leckage (Mund oder Maske undicht).

Während der Apnoe-Klassifikation mit FOT wird der vorgegebene Solldruckwert $P_{set}$ mit einer sinusförmigen Schwingung mit einer Frequenz von 2-10 Hz, beispielsweise 4 Hz überlagert.

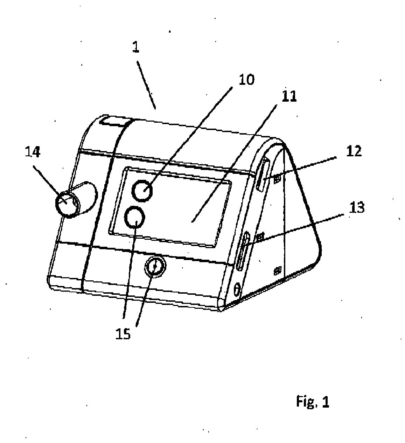

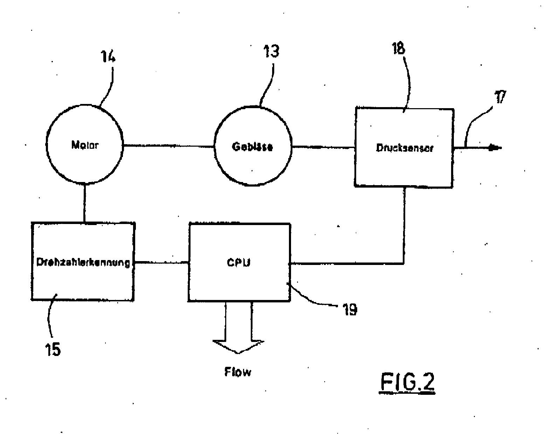

Dieser Wert (drittes Koordinatensystem in Abbildungen 1 und 2) dient dem Druckregler als Sollgrösse. Bei offenen Atemwegen muss das Gebläse mehr Luft bewegen um die vorgegebene Druckamplitude zu erreichen als im obstruktiven Fall. Damit wird eine Schwingung auf dem Fluss-Signal mit grösserer Amplitude erzeugt (vgl. oberstes Koordinatensystem in Abbildungen 1 und 2: im obstruktiven Fall hat die resultierende Fluss-Schwingung eine Amplitude von ca. 2,5 l/min im Fall der offenen Atemwege von ca. 9 l/min).

Um ein Signal für die Ausgabe im Obstruktionskanal und die Klassifizierung der Apnoen zu generieren, wird sowohl von der Druck- als auch von der Fluss-Schwingung der Maximalwert der letzten 100 Messpunkte (das entspricht einer Sekunde) bestimmt und dann der Quotient daraus berechnet:

$$FOT Quotient = \frac{FlowOscillation_{max}}{PressureOscillation_{max}}$$

Alternativ oder ergänzend kann auch rollierend für jeden Abtastschritt die Amplitude der letzten Sekunde ausgewertet werden. Alternativ oder ergänzend kann auch für jede Abtastwert das kleine Delta zum Abtastwert davor ausgewertet wird. Alternativ oder ergänzend kann auch eine Aufsummierung über 2, 3, ... Abtastschritte oder eine Verrechnung mehrerer Abtastschritte über einen Tiefpassfilter zur Glättung erfolgen.

Alternativ oder ergänzend kann auch für jeden Abtastschritt oder gemittelt über ganz wenige Abtastschritte der Quotient berechnet wird. In der Messung, die den beiden Abbildungen zugrunde liegt, beträgt der Quotient im obstruktiven Fall etwa 5, im Fall offener Atemwege liegt er bei etwa 18.

Dieser Quotient wird anschließend auf einen Wertebereich von 0 - 100 abgebildet (0 entspricht offenen Atemwegen, 100 entspricht blockierten Atemwegen). Dabei werden Effekte, die durch angeschlossenes Zubehör, ' Leckage oder Umgebungsbedingungen (Temperatur, Luftdruck) entstehen, kompensiert.

Der Quotient wird pro Abtastschritt berechnet, also ca. 100 mal pro Sekunde: Änderung Fluss versus Änderung Druck. Am Ende wird geschaut, ob während der Apnoe mehr als 50% der Zeit der Schwellwert überschritten oder unterschritten wurde.

Statt des Quotient aus Fluss und Druck könnte man auch Drehzahl und Druck, Drehzahl und Fluss etc. verwenden. Der Schwellwert des FOT könnte neben der Leckage auch je nach Zubehör angepasst werden: Befeuchter ja/nein, Bakterienfilter ja/nein, Schlauchtyp, ...

Außerdem gibt es in der Regel eine obere Grenze, bis zu der obstruktive Apnoen erkannt werden. Sie kann fix sein (bei uns 14 hPa) oder variabel je nach Druckgrenzen, als separater Einstellparameter, je nach Zubehör oder ja nach Eventverlauf des Patienten. Z.B. wenn der Patient fortlaufend bei steigenden Drücken obstruktive Apnoen zeigt, werden sie auch weiterhin als obstruktive Apnoen gewertet. Ist jedoch über einige hPa Druckanstieg keine obstruktive Apnoe mehr aufgetreten, und bei noch höheren Drücken setzen plötzlich welche ein, werden diese als reflektorische Verschlüsse aufgrund des Drucks gewertet.

Fig. 1 zeigt den grundsätzlichen Aufbau einer Vorrichtung zur Beatmung (1). Im Bereich eines Gerätegehäuses mit Bedienfeld (10) sowie Anzeige (11) ist in einem Geräteinnenraum eine Atemgaspumpe angeordnet. Über eine Kopplung (14) wird ein Verbindungsschlauch angeschlossen. Entlang des Verbindungsschlauches kann ein zusätzlicher Druckmessschlauch verlaufen, der über einen Druckeingangsstutzen mit dem Gerätegehäuse verbindbar ist. Zur Ermöglichung einer Datenübertragung weist das Gerätegehäuse zumindest eine Schnittstelle (12, 13) auf.

Im Bereich des Gerategehäuses ist ein Bedienelement (15) angeordnet, um manuell einen dynamischen Modus des Beatmungsgerätes vorgeben zu können. Das Bedienelement (15) kann im Bereich des Bedienfeldes angeordnet sein oder als externes oder separates Bedienelement ausgeführt sein.

Zur Vermeidung eines Austrocknens der Atemwege erweist es sich insbesondere bei längeren Beatmungsphasen als zweckmäßig, eine Befeuchtung der Atemluft durchzuführen. Derartige Befeuchtungen der Atemluft können auch bei anderen Anwendungen realisiert werden. Zur Befeuchtung wird seitlich ein Atemluftbefeuchter adaptiert.

Es ist möglich, dass die Steuereinrichtung dazu geeignet und ausgebildet ist, ein Flusssignal und/oder ein Drucksignal zu überwachen und anhand wenigstens eines Algorithmus auszuwerten, um ein Ereignis zu typisieren. Das Flusssignal und/oder das Drucksignal werden dabei von wenigstens einem der Steuereinrichtung zugeordneten Sensormittel bereitgestellt, Das Sensormittel ist dabei zur Erfassung wenigstens einer Flusseigenschaft und/oder wenigstens einer Druckeigenschaft des Luftstroms für die Beatmung vorgesehen. Die Auswertung von Flusssignalen bzw. Drucksignalen ermöglicht eine zuverlässige Erkennung und Typisierung von Ereignissen der Atmung.

Besonders bevorzugt ist die Steuereinrichtung auch dazu geeignet und ausgebildet, anhand des Flusssignals und/oder des Drucksignals wenigstens einen Beatmungsparameter zu registrieren, wie z.B. die Atemfrequenz, das Atemzugvolumen, das Atemminutenvolumen, den Inspirationsfluss und/oder den Inspirationsdruck und/oder den Atemwegswiderstand.

Die Steuereinrichtung kann ergänzend dazu geeignet und ausgebildet sein, den Strömungsgenerator mit einem oszillierenden Steuersignal zu beaufschlagen. Der Strömungsgenerator erzeugt daraufhin einen Atemgasfluss mit einer aufmodulieren Druckschwingung, die bevorzugt im Bereich 1 - 20 Hz, besonders bevorzugt bei 2 - 10 Hz, oder auch bei 4 Hz liegt und Druckhub von 0,1 - 1 cm H2O, bevorzugt 0,4 cmH$_2$O bewirkt. Dieser Druckhub induziert ebenfalls einen oszillierenden Fluss.

Die Steuereinrichtung kann dazu geeignet und ausgebildet sein, bei registrierten Atmungsereignissen einen entsprechenden Hinweis an einen Benutzer auszugeben. Beispielsweise kann der Hinweis mittels einer Anzeigeeinrichtung optisch und/oder akustisch angezeigt werden. Der Hinweis kann beispielsweise auch mittels einer Schnittstelle an wenigstens eine externe Datenverarbeitungseinrichtung ausgegeben werden. Ein solcher Hinweis ist besonders vorteilhaft, da in Kenntnis der Ereignisse die Beatmungsbehandlung entsprechend angepasst werden kann. Andererseits kann beim Ausbleiben des Hinweises ein entsprechender Erfolg der Beatmungsbehandlung festgestellt werden.

Das Beatmungsgerät kann dynamisch und insbesondere je nach Atemphase des Benutzers angepasst werden. Beispielsweise kann anhand der Steuereinrichtung ein Atemphasenwechsel erkannt werden, sodass je nach Atemphase ein höherer bzw. niedrigerer Druck bereitgestellt werden kann. Beispielsweise kann das Beatmungsgerät als ein CPAP- oder APAP-Gerät ausgebildet sein. Das Beatmungsgerät 1 kann auch als ein Bilevel-Gerät ausgebildet sein. Beispielsweise reagiert das Beatmungsgerät 1 auf bestimmte Atemereignisse, wie z.B. Schnarchen, Atemabflachungen und/oder obstruktive Druckspitzen mit entsprechenden Einstellungen der Beatmungsparameter.

Mit dem hier gezeigten Beatmungsgerät 1 werden Ereignisse, die in der Atmung bzw. während der Beatmung auftreten, erkannt und typisiert. Dazu erfasst das Sensormittel einen oder mehrere Beatmungsparameter wie Druck und/oder Fluss und/oder ODS-Signal und liefert entsprechende Signale an die Steuereinrichtung 7. Die Steuereinrichtung 7 analysiert die Signale mittels geeigneter Algorithmen, sodass charakteristische Signalverläufe erkannt und als ein Ereignis typisiert werden können. Dabei kann beispielsweise auf eine Parameterextraktion in Bezug auf Pegel und Amplitudenwerte, Zeitabstände, Einhüllende, Nulldurchgänge und Steigungen zurückgegriffen werden. Bei einer Analyse im Hinblick auf Zeitmerkmale wird beispielsweise bei einer Parametextraktion auf Periodizitäten und Frequenzen zurückgegriffen.

Eine obstruktive Apnoe (oA) wird erkannt, wenn für mindestens zwei Atemzüge ein stark reduziertes. Flussvolumen erkannt wird und/oder ein ODS-Anstieg erfolgt.

Eine obstruktive Hypopnoe (oH) zeigt sich beispielsweise durch ein reduziertes Flussvolumen für zwei aufeinanderfolgende Inspirationen oder durch obstruktives Flattening (eine typische Abflachung der Atemkurve) des Flusssignals.

Obstruktives Schnarchen (oS) wird durch kumuliertes Schnarchen über zumindest zwei konsekutive Inspirationen einhergehend mit mit Obstruktionen oder Flattening erkannt.

Obstruktives Flattening (oF) wird durch kumuliertes Flattening über zumindest drei Inspirationen, mindestens einen Anstieg des inspiratorischen ODS-Signals erkannt.

Ein obstruktives Ereignis (oE) wird durch einen signifikanten kumulierten inspiratorischen Anstieg des ODS-Signals erkannt.

Schnarchen (S) wird durch kumuliertes Schnarchen (zumindest 3 konsekutive Inspirationen) ohne inspiratorischen ODS-Anstieg erkannt.

Unspezifisches Flattening (uF) wird durch kumuliertes Flattening (zumindest 3 Inspirationen) erkannt.

Eine zentrale Apnoe (zA) wird über ein stark reduziertes Flussvolumen für 10 Sekunden ohne ODS-Anstieg oder Flattening erkannt.

Eine zentrale Hypopnoe (zH) wird durch ein reduziertes Flussvolumen (2 konsekutive Inspirationen) ohne ODS-Anstieg erkannt

Eine Leckage im Bereich des Patienteninterface oder eine Mund- und Maskenleckage wird dadurch erkannt, dass der Solldruck kann nicht erreicht wird und/oder der Leckagefluss über 0,6 l/sec beträgt.

Die erkannten Ereignisse können dann in der Speichereinrichtung abgelegt und für eine Beatmungsstatistik herangezogen werden. Ein Vorteil der Ereigniserkennung ist, dass auf Basis der typisierten Ereignisse eine Anpassung der Beatmung erfolgen kann, z.B. eine automatische Druckanhebung bei einer obstruktiven Apnoe. Zudem kann in einem gewissen Masse eine Diagnostik bestimmter Atemstörungen erfolgen.

Ein besonderer Vorteil des hier gezeigten Beatmungsgerätes 1 ist die Steuereinrichtung 7, welche die erkannten Ereignisse weiter analysiert und anhand eines charakteristischen Auftretens der Ereignisse auch eine CheyneStokes-Atmung registriert. Eine solche Ereignisanalyse ist beispielhaft in der Figur 2 skizziert.

Fig. 2 zeigt ein schematisches Blockschaltbild zur Erläuterung eines Teiles eines inneren Aufbaues des Gerätes entsprechend Fig. 1. Zur Förderung des Strömungsvolumens an Atemgas ist eine Pumpeinrichtung (13) vorgesehen, die als ein Gebläse ausgebildet ist. Die Pumpeinrichtung (13) wird von einem Motor (14) angetrieben. Zur Erfassung einer Drehzahl des Motors (14) ist ein Sensor (15) verwendet, der an eine Steuerung (16) angeschlossen ist. Im Bereich einer von der Pumpeinrichtung (13) gespeisten Leitung (17) für das Strömungsvolumen ist ein Sensor (18) zur Erfassung eines Druckes angeordnet. Auch der Sensor (18) ist an die Steuerung (16) angeschlossen.

Im Bereich der Steuerung (16) ist eine Recheneinheit (19) implementiert, die eine rechnerische Verknüpfung der Messwerte für den Druck und die Drehzahl vornimmt und hieraus einen aktuellen Volumenfluss berechnet. Bei einer Ausbildung der Steuerung (16) als Digitalrechner, beispielsweise als Mikroprozessor, ist es möglich, die Recheneinheit (19) als Teil der Ablaufprogramomierung der Steuerung (16) zu realisieren. Bei einer analogen Ausbildung der Steuerung (16) ist auch daran gedacht, die Recheneinheit (19) über Bauelemente mit linearem oder nichtlinearem elektrischen Verhalten zu realisieren.

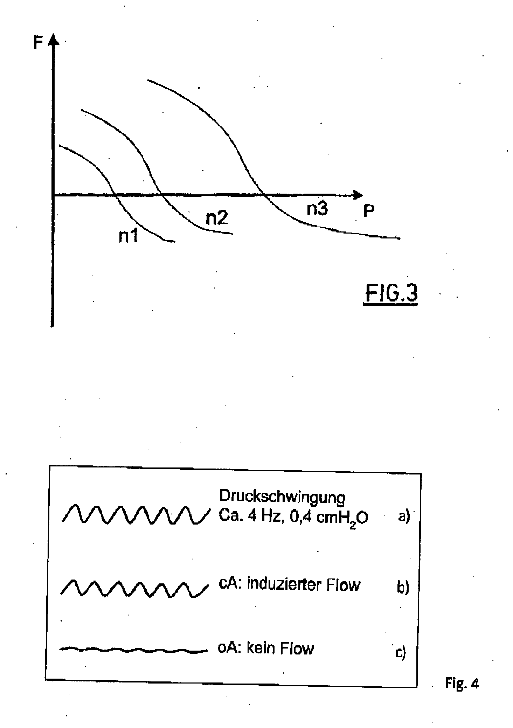

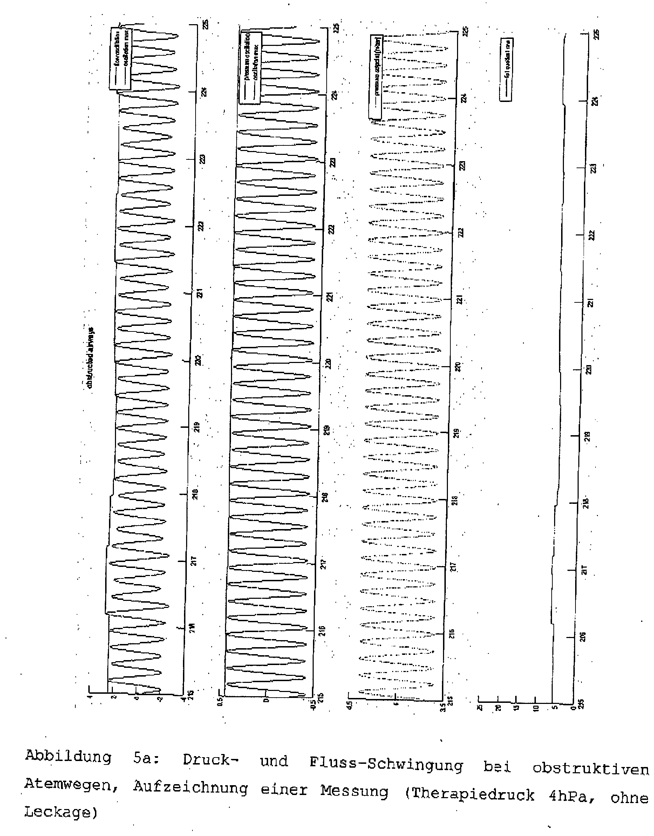

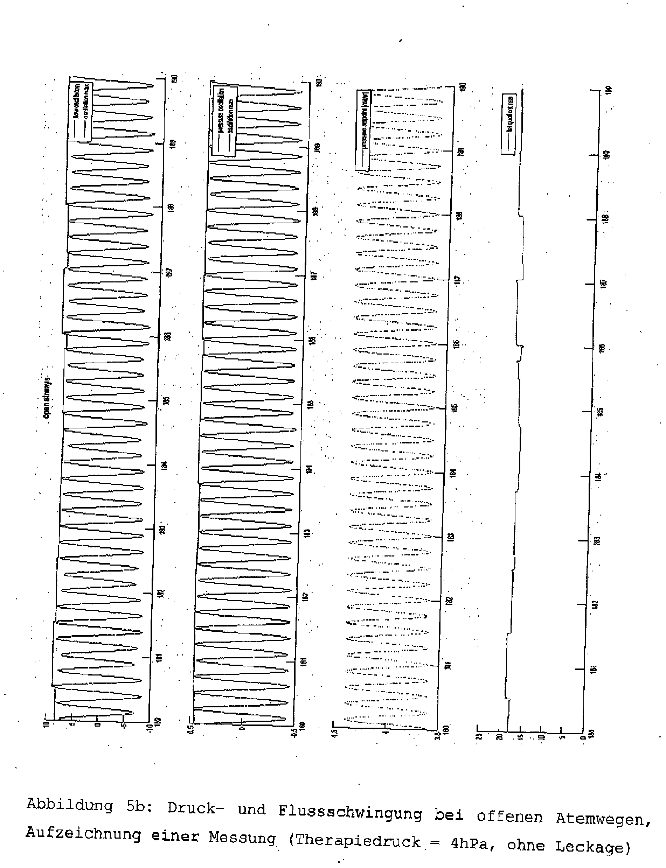

Fig. 3 veranschaulicht ein Kennlinienfeld, das für ein beispielhaft gewähltes Beatmungssystem die Abhängigkeit des Strömungsvolumens vom jeweiligen Druck wiedergibt. Es ist insbesondere zu erkennen, dass sich bei unterschiedlichen Drehzahlen des Motors (14) unterschiedliche Kennlinienverläufe ergeben. Die jeweiligen Kennlinien bestehen aus näherungsweise parabelförmigen Teilbereichen, die in einer Umgebung der Druck-Achse von näherungsweise linearen Verläufen verbunden sind.

Fig. 4 stellt einen gemessenen Verlauf (20) des Strömungsvolumens und einen von der Steuerung (16) berechneten Verlauf (21) gegenüber. Das Strömungsvolumen ist dabei in l/sec angegeben. Es ist zu erkennen, dass eine sehr weitgehende Übereinstimmung vorliegt.

Für eine Realisierung des erfindungsgemäßen Verfahrens sowie bei einem Aufbau der erfindungsgemäßen Vorrichtung wird messtechnisch das Kennlinienfeld gemäß Fig. 3 bestimmt- Für unterschiedliche Motordrehzahlen wird dabei für die Gesamtheit aus Motor (14), Pumpeinrichtung(13), Leitung (17) sowie Sensor (18) die jeweilige Kennlinie bestimmt. Für die einzelnen Kennlinien K zu jeder Drehzahl ergibt sich hierbei der Druck P in Abhängigkeit vom Volumenfluss F entsprechend der Gleichung

$$P_k(F) = a_{k2}*F^2 + a_{k1}*F + a_{k0}$$

Die obige Gleichung stellt eine Annäherung des tatsächlichen Verlaufes dar, es zeigt sich aber, dass diese Annäherung eine gute Übereinstimmung mit dem tatsächlichen Kennlinienverlauf aufweist. Jeder der Sekundärkoeffizienten $a_{ki}$ mit i = 0 bis 2 ist von der jeweiligen Drehzahl abhängig. Die obige Gleichung lässt sich deshalb auch in der Form

$$P(F) = a_2(n)*F^2 + a_1(n)*F + a_0(n)$$

darstellen. Es zeigt sich, dass auch die Sekundärkoeffizienten $a_{ki}$ in guter Näherung mit einer quadratischen Gleichung aus der jeweiligen Drehzahl bestimmt werden können. Ein entsprechender Gleichungsansatz lautet

$$a_i = b_{i2}*n^2 + b_{i1}*n + b_{i0}.$$

Die Primärkoeffizienten $b_{ik}$ repräsentieren hier die mechanischen und elektrischen Eigenschaften des Gesamtsystems und können messtechnisch bestimmt werden. Unter Berücksichtigung der quadratischen Ansätze für die Abhängigkeit zwischen dem Druck und dem Volumenfluss sowie den Sekundärkoeffizienten $a_{ik}$ von den Primärkoeffizienten $b_{ik}$ sind insgesamt neun Koeffizienten $b_{ik}$ messtechnisch zu bestimmen.

Die einfache Gleichungsstruktur ermöglicht es, die Sekundärkoeffizienten $a_{k(n)}$ vor jedem neu zu berechnenden Wert für den Volumenfluss aus den bekannten Primärkoeffizienten $b_i$ neu zu berechnen. Der eigentliche Wert für den Volumenfluss wird dann über die Umkehrfunktion der zuerst aufgeführten Gleichung ermittelt.

**Patentansprüche**

1. autoCPAP-Beatmungsvorrichtung (1), mit einer Steuereinrichtung und einem Beatmungsgebläse und einem Drucksensor, wobei die Steuereinrichtung einen Controller zur Erzeugung eines ersten Steuersignals aufweist, welches die Drehzahl des Gebläses zur Erzeugung eines unter Druck stehenden Atemgasflusses veranlasst, und wobei die Steuereinrichtung einen Controller zur Erzeugung eines periodisch veränderlichen Steuersignals aufweist, welcher das Gebläse derart ansteuert, dass die Drehzahl des Gebläses mit einer Frequenz im Bereich 1 - 20 Hz oszillierend schwankt, zur Erzeugung einer FOT-Schwingung, wobei die Steuereinrichtung eine Sensoreinrichtung aufweist, die zumindest die momentane Drehzahl des Gebläses ermittelt und/oder den momentanen elektrischen Strom des Gebläses ermittelt und/oder die momentane elektrische Leistung des Gebläses ermittelt zur Bestimmung des von dem Gebläse erzeugten Atemgasflusses und/oder Atemgasvolumens unter Verwendung von in einem Speicher hinterlegten Kenndaten des Gebläses und vom Drucksensor bereitgestellten Druckwerten **dadurch gekennzeichnet, dass**

- die autoCPAP-Beatmungsvorrichtung (1) Sensormittel zur Erfassung eines ODS-Signals aufweist und dazu ausgebildet ist, das ODS-Signal an die Steuereinrichtung zu liefern,
- die autoCPAP-Beatmungsvorrichtung (1) dazu ausgebildet ist, Ereignisse, die in der Atmung beziehungsweise während der Beatmung auftreten, zu erkennen und typisieren, wobei die Steuereinrichtung die Signale Druck und Fluss und ODS mittels geeigneter Algorithmen analysiert, sodass charakteristische Signalverlaufe erkannt und als ein Ereignis typisiert werden, wobei
- eine obstruktive Apnoe (oA) erkannt wird, wenn für mindestens zwei Atemzuge ein stark reduziertes Flussvolumen erkannt wird und/oder ein ODS-Anstieg erfolgt und wobei
- eine obstruktive Hypopnoe (oH) durch ein reduziertes Flussvolumen für zwei aufeinanderfolgende Inspirationen erkannt wird.

2. autoCPAP-Beatmungsvorrichtung (1) nach Anspruch 1 **dadurch gekennzeichnet, dass** sowohl eine Drehzahl des Motors als auch ein Druck im Bereich des Strömungsvolumens messtechnisch erfasst sowie der Steuereinrichtung zugeführt werden und dass das Strömungsvolumen von der Steuereinrichtung durch eine rechnerische Verknüpfung der Messwerte für den Druck und die Drehzahl ermittelt wird.

3. autoCPAP-Beatmungsvorrichtung (1) nach Anspruch 1 oder 2 **dadurch gekennzeichnet, dass** der Motor mit der Sensoreinrichtung zur Drehzahlerfassung versehen und im Bereich des Strömungsvolumens ein Sensor zur Druckmessung angeordnet ist, dass der Sensor und die Sensoreinrichtung an die Steuereinrichtung angeschlossen sind und dass die Steuereinrichtung mit einer Recheneinheit zur Bestimmung des Strömungsvolumens und/oder des Atemgasflusses durch eine rechnerische Verknüpfung der Messwerte für den Druck und die Drehzahl versehen ist.

4. autoCPAP-Beatmungsvorrichtung (1) nach zumindest einem der vorhergehenden Ansprüche **dadurch gekennzeichnet, dass** der ermittelte Atemgasfluss zumindest zeitweise unter Berücksichtigung der ermittelten oder abgelegten Werte zumindest eines Kompensationsparameters angepasst wird.

5. autoCPAP-Beatmungsvorrichtung (1) nach Anspruch 4 **dadurch gekennzeichnet, dass** als Kompensationsparameter Umgebungsdruck und/oder Temperatur und/oder verwendetes Zubehör und/oder die Luftfeuchte und/oder die Leckage und/oder der aktuelle Therapiedruck dienen und dass die Kompensationsparameter sensorisch ermittelt oder rechnerisch bestimmt oder ausgelesen oder vom Nutzer eingegeben werden.

6. autoCPAP-Beatmungsvorrichtung (1) nach zumindest einem der vorhergehenden Ansprüche 4 oder 5 **dadurch gekennzeichnet, dass** der ermittelte Atemgasfluss zumindest zeitweise unter Berücksichtigung der ermittelten Werte des Kompensationsparameters oder der Kompensationsparameter und unter Nutzung von Kennfeld und/oder linearer Regressionsgleichung und/oder multidimensionaler Regression und/oder Linearisierung und/oder quadratische / exponentielle Gleichungen angepasst wird.

7. autoCPAP-Beatmungsvorrichtung (1) nach zumindest einem der vorhergehenden Ansprüche **dadurch gekennzeichnet, dass** ein hinterlegter, gerätetypischer Kompensationsparameter abgerufen und genutzt wird, um den ermittelten Atemgasfluss zumindest zeitweise anzupassen.

8. autoCPAP-Beatmungsvorrichtung (1) nach zumindest einem der vorhergehenden Ansprüche **dadurch gekennzeichnet, dass** die Werte der Sensoreinrichtung noch anhand des Umgebungsdruckes korrigiert werden.

9. autoCPAP-Beatmungsvorrichtung (1) nach zumindest einem der vorhergehenden Ansprüche **dadurch gekennzeichnet, dass** die Werte der Sensoreinrichtung noch anhand der Temperatur des Atemgases am Beatmungsgebläse und/oder der Luftfeuchte korrigiert werden.

10. autoCPAP-Beatmungsvorrichtung (1) nach zumindest einem der vorhergehenden Ansprüche **dadurch gekennzeichnet, dass** die Werte der Sensoreinrichtung noch durch spezifische Kennfelder je nach Zubehörtyp korrigiert werden.

**11.** autoCPAP-Beatmungsvorrichtung (1) nach zumindest einem der vorhergehenden Ansprüche **dadurch gekennzeichnet, dass** zur Erzeugung der FOT-Schwingung der Solldruck oszillierend vom Controller geändert wird.

**12.** autoCPAP-Beatmungsvorrichtung (1) nach zumindest einem der vorhergehenden Ansprüche **dadurch gekennzeichnet, dass** insbesondere bei hohen Frequenzen der Druckregler den Druckabfall über den Schlauch und sonstiges Zubehör anhand des berechneten Atemgasflusses abschätzt und versucht diesen zu kompensieren.

**13.** autoCPAP-Beatmungsvorrichtung (1) nach zumindest einem der vorhergehenden Ansprüche **dadurch gekennzeichnet, dass** während der Apnoe-Klassifikation mit FOT der vorgegebene Solldruckwert $P_{set}$ mit einer sinusförmigen Schwingung mit einer Frequenz von 2-10 Hz, beispielsweise 4 Hz überlagert wird.

## Claims

**1.** An autoCPAP respirator (1), having a control apparatus and a respiration blower and a pressure sensor, wherein the control apparatus has a controller for generating a first control signal which induces the speed of the blower to generate a pressurized breathing gas flow, and wherein the control apparatus has a controller for generating a periodically variable control signal, which activates the blower such that the speed of the blower varies in an oscillating manner at a frequency in the range of 1 - 20 Hz in order to generate a FOT oscillation, wherein the control apparatus has a sensor apparatus which ascertains at least the instantaneous speed of the blower and/or the instantaneous electrical current of the blower and/or the instantaneous electrical power of the blower in order to determine the breathing gas flow and/or breathing gas volume generated by the blower while using characteristic data of the blower stored in a memory and pressure values provided by the pressure sensor, **characterized in that**

- the autoCPAP respirator (1) has sensor means for detecting an ODS signal and is configured to supply the ODS signal to the control apparatus,
- the autoCPAP respirator (1) is configured to identify and standardize events which occur in the breathing or respectively during the respiration, wherein the control apparatus analyzes the pressure and flow and ODS signals by means of suitable algorithms so that characteristic signal curves are identified and standardized as one event, wherein

- an obstructive apnea (oA) is identified if a significantly reduced flow volume is identified for at least two breaths and/or an ODS increase occurs and wherein
- an obstructive hypopnea (oH) is identified by a reduced flow volume for two successive inspirations.

**2.** The autoCPAP respirator (1) according to Claim 1, **characterized in that** both a speed of the motor and a pressure in the region of the flow volume are metrologically detected and supplied to the control apparatus, and the flow volume is ascertained by the control apparatus by a computed linkage of the measured values for the pressure and the speed.

**3.** The autoCPAP respirator (1) according to Claim 1 or 2, **characterized in that** the motor is provided with the sensor apparatus for speed detection and a sensor for pressure measurement is arranged in the region of the flow volume, the sensor and the sensor apparatus are connected to the control apparatus, and the control apparatus is provided with a computer unit for determining the flow volume and/or the breathing gas flow by way of a computed linkage of the measured values for the pressure and the speed.

**4.** The autoCPAP respirator (1) according to at least one of the preceding claims, **characterized in that** the ascertained breathing gas flow is adapted at least temporarily in consideration of the ascertained or stored values of at least one compensation parameter.

**5.** The autoCPAP respirator (1) according to Claim 4, **characterized in that** the ambient pressure and/or temperature and/or accessories used and/or the ambient humidity and/or the leakage and/or the current treatment pressure serve as compensation parameters, and the compensation parameters are ascertained by sensors or determined by computer or read out or input by the user.

**6.** The autoCPAP respirator (1) according to at least one of the preceding Claims 4 or 5, **characterized in that** the ascertained breathing gas flow is at least temporarily adapted in consideration of the ascertained values of the compensation parameter(s) and while using a characteristic map and/or a linear regression equation and/or a multidimensional regression and/or a linearization and/or quadratic/exponential equations.

**7.** The autoCPAP respirator (1) according to at least one of the preceding claims, **characterized in that** a stored device-typical compensation parameter is retrieved and used to adapt the ascertained breathing gas flow at least temporarily.

**8.** The autoCPAP respirator (1) according to at least one of the preceding claims, **characterized in that** the values provided by the sensor apparatus are corrected based on the ambient pressure.

**9.** The autoCPAP respirator (1) according to at least one of the preceding claims, **characterized in that** the values provided by the sensor apparatus are corrected based on the temperature of the breathing gas at the respiration blower and/or the ambient humidity.

**10.** The autoCPAP respirator (1) according to at least one of the preceding claims, **characterized in that** the values provided by the sensor apparatus are corrected by specific characteristic maps depending on accessory type.

**11.** The autoCPAP respirator (1) according to at least one of the preceding claims, **characterized in that** the target pressure is changed in an oscillating manner by the controller to generate the FOT oscillation.

**12.** The autoCPAP respirator (1) according to at least one of the preceding claims, **characterized in that** in particular at high frequencies, the pressure regulator estimates the pressure drop via the hose and other accessories on the basis of the calculated breathing gas flow and attempts to compensate for said pressure drop.

**13.** The autoCPAP respirator (1) according to at least one of the preceding claims, **characterized in that** during apnea classification with FOT, the predefined target pressure value $P_{set}$ is overlaid with a sinusoidal oscillation at a frequency of 2-10 Hz, for example 4 Hz.

**Revendications**

**1.** Dispositif de ventilation autoCPAP (1), doté d'un système de commande et d'une soufflante de ventilation et d'un capteur de pression, dans lequel le système de commande présente un contrôleur servant à générer un premier signal de commande, lequel induit la vitesse de rotation de la soufflante pour générer un flux de gaz respiratoire sous pression, et dans lequel le système de commande présente un contrôleur servant à générer un signal de commande à variations périodiques, lequel active la soufflante de telle sorte que la vitesse de rotation de la soufflante fluctue à une fréquence oscillant dans la plage de 1 - 20 Hz, servant à générer une oscillation forcée FOT, dans lequel le système de commande présente un système capteur, qui détermine au moins la vitesse de rotation momentanée de la soufflante et/ou le courant électrique momentané de la soufflante et/ou la puissance électrique momentanée de la soufflante pour définir le flux de gaz respiratoire généré par la soufflante et/ou le volume de gaz respiratoire à l'aide des caractéristiques de la soufflante enregistrées dans une mémoire et des valeurs de pression fournies par le capteur de pression, **caractérisé en ce que**

- le dispositif de ventilation autoCPAP (1) présente des moyens capteurs servant à détecter un signal de pression oscillatoire (ODS). et est conçu de manière à délivrer le signal ODS au système de commande,
- le dispositif de ventilation autoCPAP (1) est conçu de manière à dépister et à classifier des événements, qui surviennent dans la respiration ou bien durant la ventilation, dans lequel le système de commande analyse les signaux de pression, de flux et ODS moyennant des algorithmes appropriés, si bien que des courbes de variation caractéristiques des signaux sont dépistées et classifiées comme un événement, dans lequel
- une apnée obstructive (ao) est dépistée, si un flux volumétrique fortement réduit est détecté pendant au moins deux inspirations et/ou une augmentation ODS se produit et dans lequel
- une hypopnée obstructive (ho) est dépistée du fait d'un flux volumétrique réduit pendant deux inspirations consécutives.

**2.** Dispositif de ventilation autoCPAP (1) selon la revendication 1, **caractérisé en ce que** tant une vitesse de rotation du moteur qu'une pression sont détectées dans la zone du volume d'écoulement par des moyens de mesure ainsi que transférées vers le système de commande et que le volume d'écoulement est déterminé par le système de commande moyennant la corrélation mathématique des valeurs mesurées de la pression et de la vitesse de rotation.

**3.** Dispositif de ventilation autoCPAP (1) selon la revendication 1 ou 2, **caractérisé en ce que** le moteur est pourvu du système capteur pour détecter la vitesse de rotation et qu'un capteur est disposé dans la zone du volume d' écoulement pour mesurer la pression, que le capteur et le système capteur sont raccordés au système de commande et que le système de commande est pourvu d'une unité de calcul pour définir le volume d'écoulement et/ou le flux de gaz respiratoire moyennant la corrélation mathématique des valeurs mesurées de la pression et de la vitesse de rotation.

**4.** Dispositif de ventilation autoCPAP (1) selon l'une au moins des revendications précédentes, **caractérisé**

**en ce que** le flux de gaz respiratoire déterminé est adapté au moins par moments en fonction des valeurs déterminées ou enregistrées d'au moins un paramètre de compensation.

5. Dispositif de ventilation autoCPAP (1) selon la revendication 4, **caractérisé en ce que** la pression ambiante et/ou la température et/ou les accessoires utilisés et/ou l'humidité de l'air et/ou la fuite et/ou la pression thérapeutique actuelle servent de paramètres de compensation et que les paramètres de compensation sont déterminés à l'aide d'un capteur ou définis par des calculs ou bien relevés ou introduits par l'utilisateur.

6. Dispositif de ventilation autoCPAP (1) selon l'une au moins des revendications précédentes 4 ou 5, **caractérisé en ce que** le flux de gaz respiratoire déterminé est adapté au moins par moments en fonction des valeurs déterminées du paramètre de compensation ou des paramètres de compensation et par le biais d'un diagramme caractéristique et/ou d'une équation de régression linéaire et/ou d'une régression multidimensionnelle et/ou d'une linéarisation et/ou d'équations quadratiques/exponentielles.

7. Dispositif de ventilation autoCPAP (1) selon l'une au moins des revendications précédentes, **caractérisé en ce qu'**un paramètre de compensation enregistré spécifique de l'appareil est consulté et utilisé pour adapter au moins par moments le flux de gaz respiratoire déterminé.

8. Dispositif de ventilation autoCPAP (1) selon l'une au moins des revendications précédentes, **caractérisé en ce que** les valeurs du système capteur sont encore corrigées à l'aide de la pression ambiante.

9. Dispositif de ventilation autoCPAP (1) selon l'une au moins des revendications précédentes, **caractérisé en ce que** les valeurs du système capteur sont encore corrigées à l'aide de la température du gaz respiratoire au niveau de la soufflante de ventilation et/ou de l'humidité de l'air.

10. Dispositif de ventilation autoCPAP (1) selon l'une au moins des revendications précédentes, **caractérisé en ce que** les valeurs du système capteur sont encore corrigées par des diagrammes caractéristiques spécifiques suivant le type d'accessoires.

11. Dispositif de ventilation autoCPAP (1) selon l'une au moins des revendications précédentes, **caractérisé en ce que** le contrôleur fait varier la pression nominale de façon oscillante pour générer l'oscillation forcée FOT.

12. Dispositif de ventilation autoCPAP (1) selon l'une au moins des revendications précédentes, **caractérisé en ce que** le régulateur de pression évalue la chute de pression au passage du tuyau et d'autres accessoires à l'aide du flux de gaz respiratoire calculé notamment à de hautes fréquences et qu'il essaie de la compenser.

13. Dispositif de ventilation autoCPAP (1) selon l'une au moins des revendications précédentes, **caractérisé en ce que** la valeur nominale de la pression prescrite $P_{set}$ est superposée à une oscillation sinusoïdale d'une fréquence de 2-10 Hz, par exemple de 4 Hz pendant la classification de l'apnée à l'aide de la technique d'oscillation forcée FOT.

Fig. 1

FIG.2

F ↑

n1    n2    n3    P

FIG.3

Druckschwingung
Ca. 4 Hz, 0,4 cmH$_2$O    a)

cA: induzierter Flow    b)

oA: kein Flow    c)

Fig. 4

Abbildung 5a: Druck- und Fluss-Schwingung bei obstruktiven Atemwegen, Aufzeichnung einer Messung (Therapiedruck 4hPa, ohne Leckage)

Abbildung 5b: Druck- und Flussschwingung bei offenen Atemwegen, Aufzeichnung einer Messung (Therapiedruck = 4hPa, ohne Leckage)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0705615 A
- EP 0821977 A
- WO 2013042007 A
- EP 0651971 A
- WO 2008025080 A
- EP 1393767 A
- WO 2006047826 A
- WO 9710019 A
- US 2012157794 A